# DEMANDE DE BREVET EUROPEEN

(11) **EP 0 926 116 A1**
(43) Date de publication de la demande: **30.06.1999**
(21) Numéro de dépôt: 98204012.3
(22) Date de dépôt: 27.11.1998
(51) Int. Cl.: C07C 17/395, C07C 17/21, C07C 17/20, C07C 19/08, C07C 17/383

(54) **Procédés de production et d'épuration de 1,1-difluoroéthane et produit ainsi obtenu**

(30) Priorité: 01.12.1997 FR 9715265
(71) Demandeur: SOLVAY (Société Anonyme), B-1050 Bruxelles (BE)
(72) Inventeur: Anciaux, Charles-Marie, 39500 Tavaux (FR); Wilmet, Vincent, 1301 Wavre (BE); LeCroc, Dominique, 39100 Dole (FR)
(74) Mandataire: Jacques, Philippe

(57) **Abrégé**

Du 1,1-difluoroéthane contenant moins de 10 mg/kg de chlorure de vinyle est obtenu par traitement avec du fluorure d'hydrogène d'un 1,1-difluoroéthane brut contenant moins de 1 mole de chlorure d'hydrogène par mole de 1,1-difluoroéthane.

## Description

La présente invention est relative à des procédés de production et d'épuration de 1,1-difluoroéthane.

Le 1,1-difluoroéthane (HFC-152a) est par exemple destiné à être utilisé pour le gonflage des mousses plastiques (extrusion de polystyrène) ou comme agent propulseur dans les aérosols.

On connait depuis longtemps des procédés de production de 1,1-difluoroéthane par hydrofluoration de chlorure de vinyle (VC), de 1-chloro-1-fluoroéthane (HCFC-151a) ou de 1,1-dichloroéthane (HCC-150a) (voir par exemple BE-A-766395 et US-A-2.452.975).

On a cependant observé que les procédés connus conduisent généralement à un produit final encore contaminé dans une mesure inacceptable par du chlorure de vinyle.

On a par conséquent tenté de porter remède à ces inconvénients en soumettant le 1,1-difluoroéthane brut, issu de la synthèse, à une épuration. Plusieurs techniques ont été appliquées, parmi lesquelles on peut citer une hydrofluoration du produit de synthèse brut.

On peut citer en particulier un procédé d'obtention de 1,1-difluoroéthane par hydrofluoration de chlorure de vinyle en présence de SnCl₄ avec une épuration du 1,1-difluoroéthane obtenu, par distillation en présence de fluorure d'hydrogène anhydre (certificat d'auteur SU-341788). Il subsiste cependant plus de 0,6 % en chlorure de vinyle dans le HFC-152a ainsi obtenu.

On peut citer aussi la demande de brevet WO-96/40606 dans laquelle le mélange brut issu du réacteur de synthèse est introduit directement dans une étape de distillation réactive en présence de HF et éventuellement en présence d'un catalyseur tel que SnCl₄ ou BF₃. Les conditions dans la colonne de distillation sont telles qu'on obtient en tête de colonne un mélange de HFC-152a épuré et de HCI et en queue de colonne essentiellement du HF qui est recyclé au réacteur.

On comprendra aisément que, dans cette colonne dite de "distillation réactive", il se produit donc, simultanément à la distillation, une ou plusieurs réactions et que la conduite d'une telle colonne pour maintenir les conditions optimales de fonctionnement sera compliquée, surtout en présence d'un catalyseur.

D'autres techniques ont aussi été proposées pour épurer du 1,1-difluoroéthane vis-à-vis du chlorure de vinyle. On peut citer entre autre l'oxydation du chlorure de vinyle par divers oxydes (voir EP-A-0370688), une hydrogénation du chlorure de vinyle (voir WO-90/08750), une photochloration du chlorure de vinyle (voir résumé du CN-A-1074434 dans les Chemical Abstracts: 120 : 269 634 k), et une adsorption du chlorure de vinyle sur du charbon actif (EP-A-0600536).

Aucun de ces procédés ne permet toutefois d'obtenir aisément et de manière continue du 1,1-difluoroéthane contenant en permanence moins de 10 mg de VC par kg de HFC-152a.

La présente invention a pour but d'épurer du 1,1-difluoroéthane brut de façon qu'il présente invariablement une très faible teneur en chlorure de vinyle (de préférence < 10 mg/kg) et qu'il soit approprié pour les applications auxquelles ce produit est destiné. Avantageusement, ce procédé sera simple et peu compliqué à conduire.

On résout ce problème, suivant l'invention, par un procédé d'épuration de 1,1-difluoroéthane en chlorure de vinyle, comprenant un traitement par du fluorure d'hydrogène d'un 1,1-difluoroéthane brut qui se caractérise en ce que le 1,1-difluoroéthane brut présente, par mole de 1,1-difluoroéthane, une teneur en chlorure d'hydrogène (HCI) inférieure à 1 mole, avantageusement à 0,5 mole, de préférence à 0,1 mole et même à 0,03 mole. Habituellement, la teneur en chlorure de vinyle dans le 1,1-difluoroéthane à épurer varie entre environ 10 et environ 20000 mg/kg (ppm). Le procédé selon l'invention peut toutefois également être utilisé pour épurer du 1,1-difluoroéthane contenant des quantités plus importantes de VC. Généralement, il présente aussi, par kg de 1,1-difluoroéthane, une teneur en 1-chloro-1-fluoroéthane inférieure à 50 g, avantageusement à 10 g, et de préférence à 5 g, et une teneur en 1,1-dichloroéthane inférieure à 20 g, avantageusement à 5 g, et de préférence à 2 g. Par kg de HFC-152a, il contient généralement de 2 à 500 g de HF, avantageusement de 5 à 250 g, de préférence de 10 à 200 g. Un tel 1,1-difluoroéthane brut est obtenu par exemple en éliminant au moins partiellement le chlorure d'hydrogène contenu dans un produit réactionnel brut résultant de la réaction entre du fluorure d'hydrogène et un précurseur chloré du HFC-152a, tel que du chlorure de vinyle.

La présente invention concerne dès lors également un procédé de production de 1,1-difluoroéthane comprenant
a) une réaction entre du fluorure d'hydrogène et un précurseur chloré du 1,1-difluoroéthane, éventuellement en présence d'un catalyseur d'hydrofluoration, cette réaction donnant lieu à un produit réactionnel brut,
b) une séparation de chlorure d'hydrogène à partir du produit réactionnel brut, et
c) un traitement ultérieur du produit réactionnel brut, sensiblement appauvri en HCI, par du fluorure d'hydrogène, ce traitement ultérieur donnant lieu à une formation de 1,1-difluoroéthane épuré.

La suite de l'exposé est centré sur ce procédé de production de 1,1-difluoroéthane au départ d'un hydrocarbure chloré. Il est clair cependant que toutes les caractéristiques du traitement ultérieur peuvent s'appliquer également au procédé général d'épuration de 1,1-difluoroéthane mentionné plus haut.

La solution proposée par l'invention présente l'avantage de comprendre, à la sortie du réacteur de synthèse, une étape simple et courante d'appauvrissement de la teneur en chlorure d'hydrogène dans le produit de synthèse issu du réacteur principal, et une étape ultérieure dans laquelle le produit obtenu est soumis à une réaction semblable à celle qui a eu lieu dans le réacteur du synthèse, c'est-à-dire avec du fluorure d'hydrogène, de façon à convertir entièrement ou presque le chlorure de vinyle encore présent dans le produit réactionnel.

On avait constaté l'impossibilité d'atteindre une teneur en chlorure de vinyle de moins de 10 mg/kg dans le 1,1-difluoroéthane à la sortie de la réaction de synthèse, même après une distillation simple du HFC-152a produit. Dans ce dernier cas, il se forme en effet un azéotrope entre le chlorure de vinyle et le HFC-152a, ces deux composés présentant par ailleurs une très faible volatilité relative. Une distillation réactive du produit réactionnel brut tel qu'issu du réacteur, c'est-à-dire non appauvri en HCI, ne permet pas davantage d'atteindre de très faibles teneurs en chlorure de vinyle. La solution proposée suivant l'invention permet d'obvier à ces inconvénients.

Suivant un mode de réalisation préféré de l'invention, la réaction de synthèse a lieu en phase liquide dans un solvant organique. En général, on met en oeuvre du chlorure de vinyle comme précurseur chloré du 1,1-difluoroéthane. Une telle réaction a déjà été décrite dans les demandes EP-A-0637579 et EP-A-0739875. On peut aussi envisager d'alimenter un autre hydrocarbure chloré au réacteur de synthèse, en particulier du HCC-150a ou du HCFC-151a, en remplacement total ou partiel du chlorure de vinyle.

Suivant un mode de réalisation de l'invention, la séparation de HCI à partir du produit réactionnel brut issu du réacteur de synthèse s'effectue par soutirage de HCI en tète d'une colonne de distillation, dont on recueille le produit de queue pour le soumettre au traitement ultérieur.

Suivant un mode de réalisation perfectionné de l'invention, on extrait latéralement de ladite colonne de distillation un produit contenant principalement le 1,1-difluoroéthane à épurer, tandis qu'on soustrait de la queue de la colonne un produit contenant principalement des produits plus lourds que le 1,1-difluoroéthane, notamment du HF et des intermédiaires de synthèse tels que du 1,1-chlorofluoroéthane (HCFC-151a) et du 1,1-dichlornéthane (HCC-150a).

Dans ce mode de réalisation, on améliore notablement l'efficacité du traitement ultérieur en réduisant la teneur en intermédiaires de synthèse qui sont susceptibles de reformer du chlorure de vinyle pendant la réaction. On peut envisager de diminuer encore davantage cette teneur en intermédiaires de synthèse en insérant une colonne de distillation supplémentaire entre la distillation du HCI et le traitement ultérieur et en y dirigeant le produit réactionnel sensiblement appauvri en HCI. L'insertion d'une colonne de reflux entre le réacteur de synthèse et la colonne de distillation de HCI améliore aussi cette réduction en substances susceptibles de reformer du chlorure de vinyle. Dans une variante, la colonne de distillation du HCI peut ne comporter qu'un tronçon de tête, surmontant le réacteur d'hydrofluoration servant de bouilleur.

Le traitement ultérieur peut être réalisé en phase liquide ou en phase gazeuse. Avantageusement, il est effectué en phase liquide, de préférence dans un milieu réactionnel contenant au moins 200 g de HF par kg. De manière particulièrement préférée, il est réalisé dans un milieu liquide contenant au moins 500 g de HF par kg, voire au moins 800 g par kg.

Suivant un mode de réalisation avantageux de l'invention, le traitement ultérieur a lieu en présence d'un catalyseur d'hydrofluoration, qui de préférence est le même que celui utilisé dans la réaction de synthèse, si celle-ci a fait usage d'un tel catalyseur, ou en tout cas qui est utilisable dans la réaction de synthèse.

Comme catalyseurs utilisables, on peut citer les dérivés des métaux choisis parmi les métaux des groupes IIIa, IVa, IVb, Va, Vb et VIb du tableau périodique des éléments et leurs mélanges. Les dérivés de l'étain, du molybdène, du titane, du vanadium, de l'antimoine et du tungstène sont préférés. Les dérivés de l'étain sont particulièrement bien appropriés. Comme dérivés des métaux, on utilise de préférence les halogénures, tels que les chlorures, les fluorures et les chlorofluorures, ainsi que les oxydes et les oxyhalogénures. SnCl₄ est tout particulièrement préféré. En phase liquide, la quantité de catalyseur à prévoir, par litre de milieu réactionnel, peut être de l'ordre d'environ 0 à 1 mole, notamment de 0,1 à 200 mmoles, avantageusement de 0,2 à 100 mmoles et de préférence de 0,5 à 20 mmoles.

Lorsque le traitement ultérieur est réalisé dans un solvant organique, celui-ci est de préférence le même que celui de la réaction de synthèse. Comme solvant organique approprié, on peut citer notamment le perchloroéthylène ou des hydrocarbures halogénés saturés, de préférence des hydrocarbures chlorés, fluorés ou chlorofluorés contenant de 1 à 8 atomes de carbone ou leurs mélanges. En utilisant le même catalyseur, du SnCl₄ par exemple, dans la réaction de synthèse et le traitement ultérieur, on obtient une grande simplification du processus, d'autant plus que le traitement ultérieur peut s'effectuer dans des conditions analogues, notamment dans un réacteur où l'on peut bien contrôler les concentrations en catalyseur, et déterminer de manière sûre les temps de séjour nécessaires pour obtenir une réaction optimale. De plus, par le fait que le traitement ultérieur ne fait appel à aucun autre réactif ou composé chimique que ceux déjà présents dans la réaction de synthèse, il ne complique pas le reste du procédé de purification du 1,1-difluoroéthane épuré.

Le produit réactionnel brut, sensiblement appauvri en HCI, qui est mis en oeuvre dans le traitement ultérieur présente typiquement une composition similaire à celle, donnée plus haut, du 1,1-difluoroéthane brut mis en oeuvre dans le procédé général d'épuration. Si nécessaire, on peut ajouter du HF au produit réactionnel brut à épurer.

De manière avantageuse, on soumet du 1,1-difluoroéthane brut au traitement ultérieur en continu, à un débit typiquement de 0,01 à 5 kg de 1,1-difluoroéthane brut par heure et par litre de milieu réactionnel, de préférence de 0,05 à 2 kg.

Généralement, la température à laquelle le traitement ultérieur est réalisé est au moins de 40 °C et elle ne dépasse pas 130 °C. De préférence, elle est d'au moins 50 °C et elle ne dépasse pas 120 °C. La pression est choisie en fonction de la température du mélange réactionnel. Elle est généralement au moins égale à 2 bar. Le plus souvent, elle ne dépasse pas 50 bar.

La présente invention est également relative à du 1,1-difluoroéthane, obtenu par hydrofluoration d'un hydrocarbure chloré, présentant une pureté supérieure à 99,8 % en poids, de préférence supérieure à 99,9 %, et une teneur en chlorure de vinyle inférieure à 10 mg/kg, de préférence inférieure à 5 mg/kg, voire à 2 mg/kg.

D'autres traits particuliers de l'invention sont indiqués dans les revendications qui suivent.

D'autres détails et particularités de l'invention ressortiront aussi de la description de quelques exemples de réalisation donnés ci-après à titre non limitatif et avec référence aux dessins annexés.

### Exemple 1

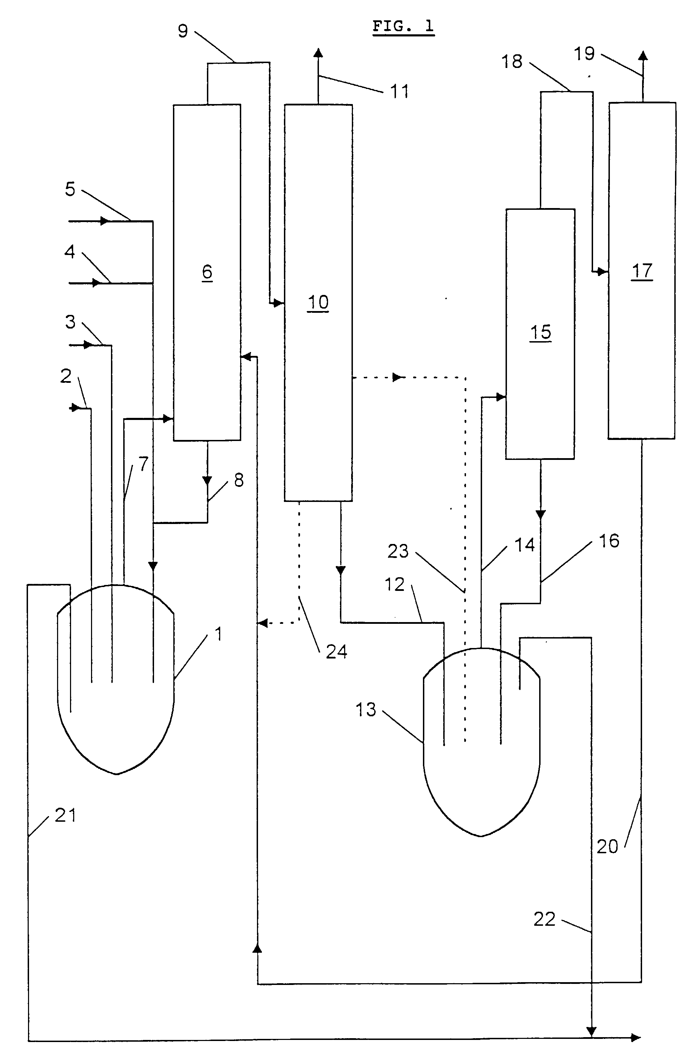

Une installation de production de 1,1-difluoroéthane suivant l'invention est illustrée de manière schématique sur la figure 1.

On peut observer sur cette figure un réacteur de synthèse 1 de 1,1-difluoroéthane, alimenté en chlorure de vinyle par un conduit d'alimentation 2 et en fluorure d'hydrogène par un autre conduit d'alimentation 3. Dans la forme de réalisation préférentielle illustrée, un catalyseur est introduit dans le réacteur 1 par un conduit d'entrée 4 qui débouche dans un conduit d'amenée 5 de solvant organique.

La réaction de synthèse s'effectue dans des conditions opératoires connues. On peut par exemple se référer ce sujet aux demandes EP-A-0637579 et EP-A-0739875.

Avantageusement on réalise dans le réacteur une température et une pression telles que le 1,1-difluoroéthane formé quitte le mélange liquide du réacteur en continu, sous la forme d'un produit réactionnel brut, gazeux, qui est amené de manière conventionnelle à une colonne de reflux 6, par l'intermédiaire du conduit 7 aménagé au sommet du réacteur 1.

Le réacteur de synthèse peut être n'importe quel réacteur connu capable de fonctionner dans les conditions de travail du procédé. On peut par exemple envisager un réacteur chauffé par un bain d'huile non représenté.

La colonne de reflux 6 fonctionne dans des conditions opératoires telles qu'elle permet de refluer au réacteur, par le conduit de retour 8 débouchant dans le conduit 5, la majeure partie des intermédiaires de synthèse HCFC-151a et HCC-150a, ainsi que du HF et d'autres composants éventuels du milieu réactionnel, notamment du solvant organique. Le produit de tête de cette colonne de reflux 6 contient typiquement environ 60 % en poids de HFC-152a, environ 30 % en poids de HCI, environ 10 % en poids de HF, environ 2 à 20 g/kg de HCFC-151 a/HCC-150a et de quelques dizaines (par exemple 20) à quelques milliers de mg (par exemple 10 000) de chlorure de vinyle par kilo.

Comme on peut le constater, ce produit de tète sortant par le conduit 9 de la colonne de reflux est encore très contaminé en chlorure de vinyle et en intermédiaires de synthèse.

Le conduit 9 débouche dans une partie intermédiaire d'une colonne de distillation 10 dont les conditions opératoires sont telles qu'on obtient en tête de colonne une séparation de HCI à l'état gazeux, qu'on évacue par le conduit de sortie 11. On soutire en queue de colonne 10 un produit réactionnel brut sensiblement appauvri en HCI, qui est amené par l'intermédiaire du conduit 12 au post-réacteur 13, dans lequel est effectué le traitement ultérieur suivant l'invention.

Le post-réacteur 13 peut être du même type que le réacteur de synthèse 1. Dans l'exemple de réalisation de l'invention illustré, le milieu réactionnel y est liquide et est composé essentiellement de fluorure d'hydrogène, avec éventuellement en supplément un solvant organique. Avantageusement, un catalyseur d'hydrofluoration se trouve dans le milieu réactionnel du post-réacteur.

Le chlorure de vinyle et les intermédiaires de synthèse HCFC-151a et HCC-150a encore contenus dans le produit de queue de la colonne de distillation 10 sont convertis en quasi totalité dans le post-réacteur 13 en HFC-152a. La teneur en chlorure de vinyle par rapport au HFC-152a quittant le post-réacteur à l'état gazeux, par le conduit 14, est typiquement inférieure à 1 mg/kg.

Dans l'exemple de réalisation illustré, on a prévu de faire passer le HFC-152a ainsi épuré en VC dans une nouvelle colonne de reflux 15, où une fraction plus lourde que cette substance est condensée et recyclée au post-réacteur 13 par conduit de retour 16.

Le produit de tête de la colonne de reflux 15 peut en supplément être conduit à une partie intermédiaire d'une colonne de distillation supplémentaire 17 par un conduit d'amenée 18.

Le HFC-152a épuré s'échappe en tête de la colonne de distillation 17 pour être amené par le conduit de sortie 19 vers des traitements supplémentaires éventuels, connus en soi, de neutralisation, de séchage et de distillation finale.

A la queue de la colonne 17, on récolte dans le conduit 20 une fraction lourde qui, dans l'exemple illustré, est ramenée vers la colonne de reflux 6.

Régulièrement des fractions lourdes sont purgées des deux réacteurs 1 et 13 et évacuées par les conduits de purge 21 et 22.

### Exemple 2

On a prévu, dans l'installation représentée sur la figure 1, une variante de réalisation qui diffère de celle décrite dans l'exemple 1 par l'abandon du conduit 12.

Dans l'installation suivant le présent exemple, un produit réactionnel brut contenant HFC-152a est extrait latéralement de la colonne de distillation 10, dans la partie inférieure de celle-ci, et est amené au post-réacteur 13 par un conduit 23 représenté en traits interrompus. Un conduit 24 agencé au pied de la colonne 10 et également représenté en traits interrompus permet de soustraire de la colonne une fraction plus lourde que le HFC-152a, en particulier des intermédiaires de synthèse de ce dernier, et de la retourner par le conduit 20 à la colonne de reflux 6.

Le produit réactionnel introduit dans le post-réacteur 13 contient donc moins d'intermédiaires de synthèse susceptibles de reformer du chlorure de vinyle en cours de réaction.

### Exemple 3

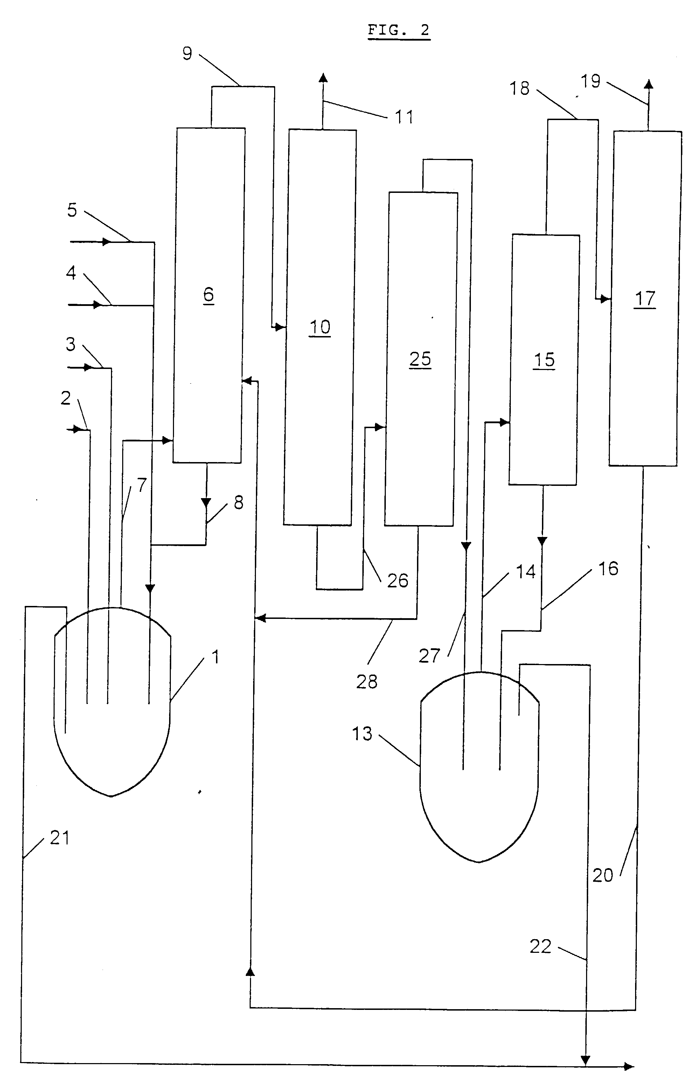

On a prévu encore une autre variante de réalisation représentée sur la figure 2.

Les parties de cette installation qui sont identiques à celles de l'installation suivant l'exemple 1 portent les mêmes références et ces parties ne seront pas décrites à nouveau.

A la différence de l'installation selon les exemples 1 et 2, le produit soutiré en pied de la colonne de distillation 10 est amené à une partie intermédiaire d'une colonne de distillation supplémentaire 25 par l'intermédiaire du conduit 26. C'est le produit de tète de cette colonne 25 qui est amené au post-réacteur 13 par l'intermédiaire du conduit 27. Le produit de queue est recyclé à la colonne de reflux 6 par le conduit de retour 28.

Cette forme de réalisation permet donc encore une amélioration supplémentaire de la réduction des intermédiaires de synthèse dans le produit amené au post-réacteur 13.

### Exemple 4

Dans une installation correspondant à celle décrite dans l'exemple 1, on a épuré dans le post-réacteur 13 du HFC-152a en chlorure de vinyle, par traitement au HF.

La composition initiale du milieu réactionnel (hors catalyseur) dans le post-réacteur 13, dans lequel le HFC-152a brut est introduit, les conditions de fonctionnement du post-réacteur et les teneurs en chlorure de vinyle avant et après le post-réacteur sont indiqués dans le tableau 1 ci-dessous.

**Tableau 1**

| Composition du milieu réactionnel initial (hors catalyseur) | Conditions de fonctionnement du post-réacteur | | | | [VC] entrée | [VC] sortie |
|---|---|---|---|---|---|---|
| | T. (°C) | P (bar) | [SnCl₄] (% poids) | Q (g/h.l) | ppm | ppm |
| HF/KFC-152a 50/50 % vol. | 65-70 | 12 | 10 | 100 | 60 | < 1 |
| HF/PER 33/66 % vol. | 88-93 | 10 | 5 | 100-150 | 1700 10 | < 1 < 1 |
| HF pur | 93-95 | 10 | 3 | 130-235 | 3000 | < 1 |
| | | | | | 1000 | < 1 |
| | | | | 200-235 | 8000 | < 1 |
| | | | | | 5000 | < 1 |
| Notes : Q = débit d'alimentation en HFC-152a brut par litre de milieu réactionnel [VC] entrée = teneur pondérale en VC dans le HFC-152a brut alimentant le post-réacteur 13 [VC] sortie = teneur pondérale en VC dans le HFC-152a traité et distillé (conduit 19) PER = perchloroéthylène [SnCl₄] = teneur pondérale en SnCl₄ par rapport au milieu réactionnel initial. | | | | | | |

### Exemple 5

Dans cet exemple on a examiné l'efficacité d'un post-réacteur 13 dans une installation selon l'exemple 1 et d'un post-réacteur 13 fonctionnant dans les mêmes conditions, dans une installation selon l'exemple 2. Le débit d'alimentation en HFC-152a brut au post-réacteur était de 0,6 kg/h.l.

Les concentrations moyennes en VC, HCFC-151a et HCC-150a, mesurées à différents endroits de l'installation sont rapportées au Tableau 2, en mg ou g par kilo de HFC-152a.

**Tableau 2**

| | VC (mg/kg) | HCFC-151a (g/kg) | HCC-150a (g/kg) |
|---|---|---|---|
| Installation Exemple 1 | | | |
| Conduit 9 | 150 | 18 | 4 |
| Conduit 19 | 1,2 | - | - |
| Installation Exemple 2 | | | |
| Conduit 9 | 140 | 15 | 4 |
| Conduit 23 | 100 | 4,5 | 0,6 |
| Conduit 19 | 0,6 | - | - |

Il ressort clairement de cet essai qu'un abaissement de la teneur en intermédiaires de synthèse du 1,1-difluoroéthane (dans le cas présent de plus de la moitié) dans le produit soumis au traitement ultérieur dans le post-réacteur 13 a pour effet de réduire drastiquement la teneur en chlorure de vinyle dans le 1,1-difluoroéthane épuré.

### Exemple 6

Dans cet exemple, une installation selon l'exemple 2 a été mise en fonctionnement avec différents débits de HFC-152a brut, de 0,6 à 0,9 kg par heure et par litre de milieu liquide au post-réacteur 13.

Les résultats obtenus sont indiqués dans le tableau 3 ci-après.

**Tableau 3**

| Débit de HFC-152a (kg/h.l) | Endroit d'échantillonnage | Concentration (par kg de HFC-152a) | | |
|---|---|---|---|---|
| | | VC (mg/kg) | HCFC-151a (g/kg) | HCC-150a (g/kg) |
| 0,6 | Conduit 9 | 30 | 4,4 | 0,75 |
| | Conduit 23 | 23 | 2,4 | 0,3 |
| | Conduit 19 | 0,6 | - | - |
| 0,7 | Conduit 9 | 45 | 7,3 | 1,5 |
| | Conduit 23 | 42 | 4 | 0,6 |
| | Conduit 19 | 0,6 | - | - |
| 0,8 | Conduit 9 | 43 | 8 | 1,8 |
| | Conduit 23 | 41 | 3,9 | 0,6 |
| | Conduit 19 | 0,7 | - | - |
| 0,9 | Conduit 9 | 52 | 11,7 | 2,9 |
| | Conduit 23 | 53 | 6,8 | 1,1 |
| | Conduit 19 | 0,9 | - | - |

Il ressort de ce tableau que le procédé d'épuration reste efficace même pour des débits élevés de production.

Il doit être entendu que la présente invention n'est pas limitée aux exemples de réalisation décrits ci-dessus et que bien des modifications peuvent y être apportées sans sortir du cadre des revendications annexées.

## Revendications

1. Procédé d'épuration de 1,1-difluoroéthane en chlorure de vinyle, comprenant un traitement par du fluorure d'hydrogène d'un 1,1-difluoroéthane brut contenant moins de 1 mole de chlorure d'hydrogène par mole de 1,1-difluoroéthane.

2. Procédé suivant la revendication 1, dans lequel le 1,1-difluoroéthane brut présente, par kg de 1,1-difluoroéthane, une teneur en 1-chloro-1-fluoroéthane inférieure à 50 g et une teneur en 1,1-dichloroéthane inférieure à 20 g.

3. Procédé suivant la revendication 1 ou 2, dans lequel le traitement est réalisé en phase liquide dans un milieu réactionnel contenant au moins 200 g de HF par kg, de préférence au moins 500 g de HF par kg.

4. Procédé suivant une quelconque des revendications 1 à 3, dans lequel le traitement est effectué en présence d'un catalyseur d'hydrofluoration choisi parmi le groupe comprenant des dérivés des groupes IIIa, IVa, IVb, Va, Vb et VIb du tableau périodique des éléments et leurs mélanges, de préférence parmi les dérivés de l'étain, du molybdène, du titane, du vanadium, de l'antimoine et du tungstène.

5. Procédé suivant l'une quelconque des revendications 1 à 4, selon lequel on soumet le 1,1-difluoroéthane brut audit traitement en continu, à un débit de 0,01 à 5 kg par heure et par litre de milieu réactionnel.

6. Procédé de production de 1,1-difluoroéthane comprenant
a) une réaction entre du fluorure d'hydrogène et un précurseur chloré du 1,1-difluoroéthane, éventuellement en présence d'un catalyseur d'hydrofluoration, cette réaction donnant lieu à un produit réactionnel brut;
b) une séparation de chlorure d'hydrogène à partir du produit réactionnel brut; et
c) un traitement ultérieur du produit réactionnel brut, sensiblement appauvri en HCI, par du fluorure d'hydrogène, ce traitement ultérieur donnant lieu à une formation de 1,1-difluoroéthane épuré.

7. Procédé suivant la revendication 6, caractérisé en ce que la séparation de HCI est effectuée dans une colonne de distillation avec élimination de HCI en tête de colonne et soutirage en queue de colonne du produit réactionnel brut, sensiblement appauvri en HCI, destiné au traitement ultérieur.

8. Procédé suivant la revendication 6, caractérisé en ce que la séparation de HCI est effectuée dans une colonne de distillation avec élimination de HCI en tête de colonne, extraction latérale du produit réactionnel brut sensiblement appauvri en HCl, destiné au traitement ultérieur, et soutirage d'un produit de queue contenant des produits plus lourds que le 1,1-difluoroéthane, lequel produit de queue est éventuellement renvoyé à l'étape a).

9. Procédé suivant l'une ou l'autre des revendications 7 et 8, caractérisé en ce qu'il comprend une distillation supplémentaire dudit produit réactionnel brut sensiblement appauvri en HCI, provenant de ladite colonne de distillation de HCI, avec soutirage en tête de colonne du produit destiné au traitement ultérieur, et soutirage en queue de colonne d'un produit de queue contenant des produits plus lourds que le 1,1-difluoroéthane, lequel produit de queue est éventuellement renvoyé à l'étape a).

10. 1,1-Difluoroéthane, obtenu par hydrofluoration d'un hydrocarbure chloré, présentant une pureté supérieure à 99,8 %, de préférence supérieure à 99,9 %, et une teneur en chlorure de vinyle inférieure à 10 mg/kg, de préférence inférieure à 5 mg/kg.
